# EUROPEAN PATENT APPLICATION

(11) **EP 1 577 395 A1**
(43) Date of publication of application: **21.09.2005**
(21) Application number: 03767379.5
(22) Date of filing: 19.12.2003
(51) Int. Cl.: C12N 15/82

(54) **ARTIFICIAL PROMOTER FOR THE EXPRESSION OF DNA SEQUENCES IN VEGETAL CELLS**

(30) Priority: 27.12.2002 CU 33702
(71) Applicant: CENTRO DE INGENIERIA GENETICA Y BIOTECNOLOGIA, Ciudad de La Habana 10600 (CU)
(72) Inventor: SELMAN-HOUSEIN SOSA, Guillermo, 12100 Ciudad la la Habana (CU); SALAZAR RODR GUEZ, Alberto, 11100 Ciudad de la Habana (CU); ABREU REMEDIOS, Daymi, 60200 Sancti Spiritus (CU); RAMOS GONZ LEZ, Osmany, 13500 Ciudad de la Habana (CU)
(74) Representative: Winckels, Johannes Hubertus F.
(86) International application number: PCT/CU2003/000018
(87) International publication number: WO 2004/058979

(57) **Abstract**

An artificial promoter **characterized** for been a chimerical recombinant DNA molecule such that, when introduced in any class of plant cells, promotes high expression levels of any DNA molecule fused to its 3' end. The basic genetic elements of the molecule described here are: a core promoter with a consensus TATA box, followed by an Exon/Intron/Exon region and a translation enhancer element, all of them artificially constructed. Transcription regulatory elements can be inserted upstream of the promoter here described to confer temporal-, organ- or tissue-specificity to the expression. The designed artificial genetic elements can be functionally inserted between any promoter active in plant cells and any DNA sequence to increase its transcription/translation levels.

SEQUENCE LISTING

<110> Centro de Ingeniería Genética y Biotecnología

<120> Artificial promoter for the expression of DNA sequences in plant
cells

<130> Artificial promoter

<140> 0000
<141> 2002-11-18

<160> 22

<170> PatentIn Ver. 2.1

<210> 1
<211> 86
<212> DNA
<213> Artificial sequence

<220>
<223> Artificial sequence description:
      Translational enhancer Eureka.

<400> 1
gaaacaaatt gaacatcatt ctatcaatac aacacaaaca caacacaact caatcattta 60
tttgacaaca caactaaaca accatg                                       86


<210> 2
<211> 198
<212> DNA
<213>

<220>
<223> Artificial sequence description:
      Synthetic fragment P35AcU.

<400> 2
gaattctata tataggaagt tcatttcatt tggagccccc caaccctacc accaccacca 60
ccaccacctc ctccttcaca caacacacac acaacagatc tcccccatcc tccctcccgt 120
cgcgccgcgc aacacctggt aagatggctg tgcgctcaga tatatatagt gatatgcact 180
acaaagatca taactagt                                                198


<210> 3
<211> 231
<212> DNA
<213>

<220>
<223> Artificial sequence description:
      Synthetic fragment I-U/Ac.

<400> 3
ctagaccgcc gcctcccccc ccccccctct ctaccttctc tctttctttc tccgtttttt 60
ttttccgtct cgtctcgatc tttggccttg gtagtttggg ggcgagaggc ggcttcgtcg 120
cccagatcgg tgcgcgtttt tttatttgga ggggcgggat ctcgcggctg ggtctcggcg 180
tgcggccgga ttctcgcggg gaatggggct ctcggatgtg gatccgagct c          231

<210> 4
<211> 255
<212> DNA
<213> Artificial sequence

<220>
<223> Artificial sequence description:
      Synthetic fragment I-Ac/U.

<400> 4
gatctgatcc gccgttgttg ggggagatat ggggcgttta aaatttcgcc atgctaaaca 60
agatcaggaa gaggggaaaa gggcactatg gtttaatttt tatatatttc tgctgctgct 120
cgtcaggatt agatgtgctt gatctttctt tcttcttttt gtgggtagaa tttgaatccc 180
tcagcattgt tcatcggtag tttttctttt gtcgatgctc accctgttgt ttggtgtttt 240
tatactagtg agctc 255


<210> 5
<211> 93
<212> DNA
<213> Artificial sequence

<220>
<223> Artificial sequence Description:
      Synthetic fragment IniT.

<400> 5
ctagtggcta tcctgacacg gtctctttgt caaatatctc tgtgtgcagg tataactgca 60
ggaaacaaca acaataacca tggtctagag ctc 93


<210> 6
<211> 692
<212> DNA
<213>

<220>
<223> Artificial sequence description:
      Artificial Exon/Intron/Exon ART.

<400> 6
accaccacca ccaccaccac ctcctccttc acacaacaca cacacaacag atctccccca 60
tcctccctcc cgtcgcgccg cgcaacacct ggtaagatgg ctgtgcgctc agatatatat 120
agtgatatgc actacaaaga tcataactag accgccgcct cccccccccc ccctctctac 180
cttctctctt tctttctccg tttttttttt ccgtctcgtc tcgatctttg gccttggtag 240
tttgggggcg agaggcggct tcgtcgccca gatcggtgcg cgttttttta tttggagggg 300
cgggatctcg cggctgggtc tcggcgtgcg gccggattct cgcggggaat ggggctctcg 360
gatgtggatc tgatccgccg ttgttggggg agatatgggg cgtttaaaat ttcgccatgc 420
taaacaagat caggaagagg ggaaaagggc actatggttt aatttttata tatttctgct 480
gctgctcgtc aggattagat gtgcttgatc tttctttctt ctttttgtgg gtagaatttg 540
aatccctcag cattgttcat cggtagtttt tcttttgtcg atgctcaccc tgttgtttgg 600
tgtttttata ctagtggcta tcctgacacg gtctctttgt caaatatctc tgtgtgcagg 660
tataactgca ggaaacaaca acaataacca tg 692


<210> 7
<211> 750
<212> DNA
<213>

<220>
<223> Artificial sequence description:
      pBS-ART vector sequence between the restriction sites EcoRI and
SacI.

<400> 7
gaattctata tataggaagt tcatttcatt tggagccccc caaccctacc accaccacca 60
ccaccacctc ctccttcaca caacacacac acaacagatc tcccccatcc tccctcccgt 120
cgcgccgcgc aacacctggt aagatggctg tgcgctcaga tatatatagt gatatgcact 180
acaaagatca taactagacc gccgcctccc cccccccccc tctctacctt ctctctttct 240
ttctccgttt tttttttccg tctcgtctcg atctttggcc ttggtagttt gggggcgaga 300
ggcggcttcg tcgcccagat cggtgcgcgt ttttttattt ggaggggcgg gatctcgcgg 360
ctgggtctcg gcgtgcggcc ggattctcgc ggggaatggg gctctcggat gtggatctga 420
tccgccgttg ttgggggaga tatggggcgt ttaaaatttc gccatgctaa acaagatcag 480
gaagagggga aaagggcact atggtttaat ttttatatat ttctgctgct gctcgtcagg 540
attagatgtg cttgatcttt ctttcttctt tttgtgggta gaatttgaat ccctcagcat 600
tgttcatcgg tagtttttct tttgtcgatg ctcaccctgt tgtttggtgt ttttatacta 660
gtggctatcc tgacacggtc tctttgtcaa atatctctgt gtgcaggtat aactgcagga 720
aacaacaaca ataaccatgg tctagagctc 750


<210> 8
<211> 757
<212> DNA
<213> Artificial sequence


<220>
<223> Artificial sequence description:
      Artificial Exon/Intron/Exon ARTE.

<400> 8
accaccacca ccaccaccac ctcctccttc acacaacaca cacacaacag atctccccca 60
tcctccctcc cgtcgcgccg cgcaacacct ggtaagatgg ctgtgcgctc agatatatat 120
agtgatatgc actacaaaga tcataactag accgccgcct cccccccccc ccctctctac 180
cttctctctt tctttctccg tttttttttt ccgtctcgtc tcgatctttg gccttggtag 240
tttgggggcg agaggcggct tcgtcgccca gatcggtgcg cgttttttta tttggagggg 300
cgggatctcg cggctgggtc tcggcgtgcg gccggattct cgcggggaat ggggctctcg 360
gatgtggatc tgatccgccg ttgttggggg agatatgggg cgtttaaaat ttcgccatgc 420
taaacaagat caggaagagg ggaaaagggc actatggttt aatttttata tatttctgct 480
gctgctcgtc aggattagat gtgcttgatc tttctttctt ctttttgtgg gtagaatttg 540
aatccctcag cattgttcat cggtagtttt tcttttgtcg atgctcaccc tgttgtttgg 600
tgtttttata ctagtggcta tcctgacacg gtctctttgt caaatatctc tgtgtgcagg 660
tataactgca ggaaacaaat tgaacatcat tctatcaata caacacaaac acaacacaac 720
tcaatcattt atttgacaac acaactaaac aaccatg 757


<210> 9
<211> 815
<212> DNA
<213> Artificial sequence

<220>
<223> Artificial sequence description:
      pPARTE vector sequence between the restriction sites EcoRI and
SacI.

<400> 9
gaattctata tataggaagt tcatttcatt tggagccccc caaccctacc accaccacca 60
ccaccacctc ctccttcaca caacacacac acaacagatc tcccccatcc tccctcccgt 120

cgcgccgcgc aacacctggt aagatggctg tgcgctcaga tatatatagt gatatgcact 180
acaaagatca taactagacc gccgcctccc cccccccccc tctctacctt ctctctttct 240
ttctccgttt tttttttccg tctcgtctcg atctttggcc ttggtagttt gggggcgaga 300
ggcggcttcg tcgcccagat cggtgcgcgt ttttttattt ggaggggcgg gatctcgcgg 360
ctgggtctcg gcgtgcggcc ggattctcgc ggggaatggg gctctcggat gtggatctga 420
tccgccgttg ttgggggaga tatggggcgt ttaaaatttc gccatgctaa acaagatcag 480
gaagagggga aaagggcact atggtttaat ttttatatat ttctgctgct gctcgtcagg 540
attagatgtg cttgatcttt ctttcttctt tttgtgggta gaatttgaat ccctcagcat 600
tgttcatcgg tagtttttct tttgtcgatg ctcaccctgt tgtttggtgt ttttatacta 660
gtggctatcc tgacacggtc tctttgtcaa atatctctgt gtgcaggtat aactgcagga 720
aacaaattga acatcattct atcaatacaa cacaaacaca acacaactca atcatttatt 780
tgacaacaca actaaacaac catggtctag agctc 815

<210> 10
<211> 184
<212> DNA
<213> Artificial sequence

<220>
<223> Artificial sequence description:
      Synthetic fragment En-Ac1.

<400> 10
atcaccgtga gttgtccgca ccaccgcacg tctcgcagcc aaaaaaaaaa aaagaaagaa 60
aaaaaagaaa aagaaaaaac agcaggtggg tccgggtcgt gggggccgga aaagcgagga 120
ggatcgcgag cagcgacgag gccggccctc cctccgcttc caaagaaacg ccccccatca 180
attc 184

<210> 11
<211> 94
<212> DNA
<213> Artificial sequence

<220>
<223> Artificial sequence description:
      Synthetic fragment En-Ac2.

<400> 11
aagcttgata tccatagcaa gcccagccca acccaaccca acccaaccca ccccagtgca 60
gccaactggc aaatagtctc cacaccccgg cact 94

<210> 12
<211> 1087
<212> DNA
<213> Artificial sequence

<220>
<223> Artificial sequence description:
      pAPARTE vector sequence between the restriction sites HindIII y
SacI.

<400> 12
aagcttgata tccatagcaa gcccagccca acccaaccca acccaaccca ccccagtgca 60
gccaactggc aaatagtctc cacaccccgg cactatcacc gtgagttgtc cgcaccaccg 120
cacgtctcgc agccaaaaaa aaaaaaagaa agaaaaaaaa gaaaaagaaa aaacagcagg 180
tgggtccggg tcgtgggggc cggaaaagcg aggaggatcg cgagcagcga cgaggccggc 240
cctccctccg cttccaaaga aacgcccccc atcaattcta tatataggaa gttcatttca 300
tttggagccc cccaacccta ccaccaccac caccaccacc tcctccttca cacaacacac 360

acacaacaga tctcccccat cctccctccc gtcgcgccgc gcaacacctg gtaagatggc 420
tgtgcgctca gatatatata gtgatatgca ctacaaagat cataactaga ccgccgcctc 480
cccccccccc cctctctacc ttctctcttt ctttctccgt tttttttttc cgtctcgtct 540
cgatctttgg ccttggtagt ttgggggcga gaggcggctt cgtcgcccag atcggtgcgc 600
gtttttttat ttggaggggc gggatctcgc ggctgggtct cggcgtgcgg ccggattctc 660
gcggggaatg gggctctcgg atgtggatct gatccgccgt tgttggggga gatatggggc 720
gtttaaaatt tcgccatgct aaacaagatc aggaagaggg gaaaagggca ctatggttta 780
atttttatat atttctgctg ctgctcgtca ggattagatg tgcttgatct ttctttcttc 840
tttttgtggg tagaatttga atccctcagc attgttcatc ggtagttttt cttttgtcga 900
tgctcaccct gttgtttggt gtttttatac tagtggctat cctgacacgg tctctttgtc 960
aaatatctct gtgtgcaggt ataactgcag gaaacaaatt gaacatcatt ctatcaatac 1020
aacacaaaca caacacaact caatcattta tttgacaaca caactaaaca accatggtct 1080
agagctc 1087

<210> 13
<211> 31
<212> ADN
<213> Artificial sequence

<220>
<223> Artificial sequence description:
      Synthetic fragment ASP.

<400> 13
gtcgactgac gcttcgaatg acgcacatgc c 31

<210> 14
<211> 1065
<212> DNA
<213> Artificial sequence

<220>
<223> Artificial sequence description:
      p2A1PARTE vector between the restriction sites KpnI and SacI.

<400> 14
ggtaccgggc cccccctcga ctgacgcttc gaatgacgca catgccatca ccgtgagttg 60
tccgcaccac cgcacgtctc gcagccaaaa aaaaaaaaag aaagaaaaaa aagaaaaaga 120
aaaaacagca ggtgggtccg ggtcgtgggg gccggaaaag cgaggaggat cgctgacgct 180
tcgaatgacg cacatgcccg agcagcgacg aggccggccc tccctccgct tccaaagaaa 240
cgccccccat caattctata tataggaagt tcatttcatt tggagccccc caaccctacc 300
accaccacca ccaccacctc ctccttcaca caacacacac acaacagatc tcccccatcc 360
tccctcccgt cgcgccgcgc aacacctggt aagatggctg tgcgctcaga tatatatagt 420
gatatgcact acaaagatca taactagacc gccgcctccc cccccccccc tctctacctt 480
ctctctttct ttctccgttt tttttttccg tctcgtctcg atctttggcc ttggtagttt 540
gggggcgaga ggcggcttcg tcgcccagat cggtgcgcgt ttttttattt ggaggggcgg 600
gatctcgcgg ctgggtctcg gcgtgcggcc ggattctcgc ggggaatggg gctctcggat 660
gtggatctga tccgccgttg ttgggggaga tatggggcgt ttaaaatttc gccatgctaa 720
acaagatcag gaagagggga aaagggcact atggtttaat ttttatatat ttctgctgct 780
gctcgtcagg attagatgtg cttgatcttt ctttcttctt tttgtgggta gaatttgaat 840
ccctcagcat tgttcatcgg tagtttttct tttgtcgatg ctcaccctgt tgtttggtgt 900
ttttatacta gtggctatcc tgacacggtc tctttgtcaa atatctctgt gtgcaggtat 960
aactgcagga aacaaattga acatcattct atcaatacaa cacaaacaca acacaactca 1020
atcatttatt tgacaacaca actaaacaac catggtctag agctc 1065

<210> 15
<211> 1135
<212> DNA

<213> Artificial sequence

<220>
<223> Artificial sequence description:
      p2APARTE vector sequence between the restriction sites SalI and
SacI.

<400> 15
gtcgactgac gcttcgaatg acgcacatgc catccatagc aagcccagcc caacccaacc 60
caacccaacc caccccagtg cagccaactg gcaaatagtc tccacacccc ggcactatca 120
ccgtgagttg tccgcaccac cgcacgtctc gcagccaaaa aaaaaaaaag aaagaaaaaa 180
aagaaaaaga aaaaacagca ggtgggtccg ggtcgtgggg gccggaaaag cgaggaggat 240
cgctgacgct tcgaatgacg cacatgcccg agcagcgacg aggccggccc tccctccgct 300
tccaaagaaa cgccccccat caattctata tataggaagt tcatttcatt tggagccccc 360
caaccctacc accaccacca ccaccacctc ctccttcaca caacacacac acaacagatc 420
tcccccatcc tccctcccgt cgcgccgcgc aacacctggt aagatggctg tgcgctcaga 480
tatatatagt gatatgcact acaaagatca taactagacc gccgcctccc cccccccccc 540
tctctacctt ctctctttct ttctccgttt tttttttccg tctcgtctcg atctttggcc 600
ttggtagttt gggggcgaga ggcggcttcg tcgcccagat cggtgcgcgt ttttttattt 660
ggaggggcgg gatctcgcgg ctgggtctcg gcgtgcggcc ggattctcgc ggggaatggg 720
gctctcggat gtggatctga tccgccgttg ttgggggaga tatggggcgt ttaaaatttc 780
gccatgctaa acaagatcag gaagagggga aaagggcact atggtttaat ttttatatat 840
ttctgctgct gctcgtcagg attagatgtg cttgatcttt ctttcttctt tttgtgggta 900
gaatttgaat ccctcagcat tgttcatcgg tagtttttct tttgtcgatg ctcaccctgt 960
tgtttggtgt ttttatacta gtggctatcc tgacacggtc tctttgtcaa atatctctgt 1020
gtgcaggtat aactgcagga aacaaattga acatcattct atcaatacaa cacaaacaca 1080
acacaactca atcatttatt tgacaacaca actaaacaac catggtctag agctc 1135


<210> 16
<211> 31
<212> DNA
<213> Artificial sequence

<220>
<223> Artificial sequence description:
      1.

<400> 16
gaaggtaccg ccatggtcta aaggacaatt g 31


<210> 17
<211> 27
<212> DNA
<213> Artificial sequence

<220>
<223> Artificial sequence description:
      Oligonucleotidic primer Oli-U2.

<400> 17
ctcctcgagg gcgtttaaca ggctggc 27


<210> 18
<211> 186
<212> DNA
<213> Artificial sequence

<220>

<223> Artificial sequence description:
      Synthetic fragment En-U2.

<400> 18
ggtaccgagc attgcatgtc taagttataa aaaattacca catatttttt ttgtcacact 60
tgtttgaagt gcagtttatc tatctttata catatattta aactttactc tacgaataat 120
ataatctata gtacaacaat aatatcagtg ttttagagaa tcatataaat gaacagttag 180
acatgg                                                            186


<210> 19
<211> 563
<212> DNA
<213> Artificial sequence

<220>
<223> Artificial sequence description:
      Maize ubiquitine-1 gene derived transcriptional enhancer sequence
(region from -299 a -855).

<400> 19
ggtaccgagc attgcatgtc taagttataa aaaattacca catatttttt ttgtcacact 60
tgtttgaagt gcagtttatc tatctttata catatattta aactttactc tacgaataat 120
ataatctata gtacaacaat aatatcagtg ttttagagaa tcatataaat gaacagttag 180
acatggtcta aaggacaatt gagtattttg acaacaggac tctacagttt tatcttttta 240
gtgtgcatgt gttctccttt ttttttgcaa atagcttcac ctatataata cttcatccat 300
tttattagta catccattta gggtttaggg ttaatggttt ttatagacta atttttttag 360
tacatctatt ttattctatt ttagcctcta aattaagaaa actaaaactc tattttagtt 420
tttttattta ataatttaga tataaaatag aataaaataa agtgactaaa aattaaacaa 480
atacccttta agaaattaaa aaaactaagg aaacattttt cttgtttcga gtagataatg 540
ccagcctgtt aaacgccctc gac                                         563


<210> 20
<211> 1692
<212> DNA
<213> Artificial sequence

Secuencia <220>
<223> Artificial sequence description:
      pU3ARTE vector sequence between the restriction sites KpnI and
SacI.

<400> 20
ggtaccgagc attgcatgtc taagttataa aaaattacca catatttttt ttgtcacact 60
tgtttgaagt gcagtttatc tatctttata catatattta aactttactc tacgaataat 120
ataatctata gtacaacaat aatatcagtg ttttagagaa tcatataaat gaacagttag 180
acatggtcta aaggacaatt gagtattttg acaacaggac tctacagttt tatcttttta 240
gtgtgcatgt gttctccttt ttttttgcaa atagcttcac ctatataata cttcatccat 300
tttattagta catccattta gggtttaggg ttaatggttt ttatagacta atttttttag 360
tacatctatt ttattctatt ttagcctcta aattaagaaa actaaaactc tattttagtt 420
tttttattta ataatttaga tataaaatag aataaaataa agtgactaaa aattaaacaa 480
atacccttta agaaattaaa aaaactaagg aaacattttt cttgtttcga gtagataatg 540
ccagcctgtt aaacgccctc gactgacgct tcgaatgacg cacatgccat ccatagcaag 600
cccagcccaa cccaacccaa cccaacccac cccagtgcag ccaactggca aatagtctcc 660
acaccccggc actatcaccg tgagttgtcc gcaccaccgc acgtctcgca gccaaaaaaa 720
aaaaaagaaa gaaaaaaaag aaaaagaaaa aacagcaggt gggtccgggt cgtgggggcc 780
ggaaaagcga ggaggatcgc tgacgcttcg aatgacgcac atgcccgagc agcgacgagg 840
ccggccctcc ctccgcttcc aaagaaacgc cccccatcaa ttctatatat aggaagttca 900
tttcatttgg agccccccaa ccctaccacc accaccacca ccacctcctc cttcacacaa 960
cacacacaca acagatctcc cccatcctcc ctcccgtcgc gccgcgcaac acctggtaag 1020

atggctgtgc gctcagatat atatagtgat atgcactaca aagatcataa ctagaccgcc 1080
gcctcccccc ccccccctct ctaccttctc tctttctttc tccgtttttt ttttccgtct 1140
cgtctcgatc tttggccttg gtagtttggg ggcgagaggc ggcttcgtcg cccagatcgg 1200
tgcgcgtttt tttatttgga ggggcgggat ctcgcggctg ggtctcggcg tgcggccgga 1260
ttctcgcggg gaatggggct ctcggatgtg gatctgatcc gccgttgttg ggggagatat 1320
ggggcgttta aaatttcgcc atgctaaaca agatcaggaa gaggggaaaa gggcactatg 1380
gtttaatttt tatatatttc tgctgctgct cgtcaggatt agatgtgctt gatctttctt 1440
tcttcttttt gtgggtagaa tttgaatccc tcagcattgt tcatcggtag tttttctttt 1500
gtcgatgctc accctgttgt ttggtgtttt tatactagtg gctatcctga cacggtctct 1560
ttgtcaaata tctctgtgtg caggtataac tgcaggaaac aaattgaaca tcattctatc 1620
aatacaacac aaacacaaca caactcaatc atttatttga caacacaact aaacaaccat 1680
ggtctagagc tc 1692

<210> 21
<211> 223
<212> DNA
<213> Artificial sequence

<220>
<223> Artificial sequence description:
      Synthetic fragment GLU.

<400> 21
ctcgagatac atattaagag tatggacaga catttcttta acaaactcca tttgtattac 60
tccaaaagca ccagaagttt gtcatggctg agtcatgaaa tgtatagttc aatcttgcaa 120
agttgccttt ccttttgtac tgtgttttaa cactacaagc catatattgt ctgtacgtgc 180
aacaaactat atcaccatgt atcccaagat gcttttttaa ttc 223

<210> 22
<211> 1032
<212> DNA
<213> Artificial sequence

<220>
<223> Artificial sequence description:
      pGARTE vector sequence between the restriction sites XhoI and SacI.

<400> 22
ctcgagatac atattaagag tatggacaga catttcttta acaaactcca tttgtattac 60
tccaaaagca ccagaagttt gtcatggctg agtcatgaaa tgtatagttc aatcttgcaa 120
agttgccttt ccttttgtac tgtgttttaa cactacaagc catatattgt ctgtacgtgc 180
aacaaactat atcaccatgt atcccaagat gcttttttaa ttctatatat aggaagttca 240
tttcatttgg agccccccaa ccctaccacc accaccacca ccacctcctc cttcacacaa 300
cacacacaca acagatctcc cccatcctcc ctcccgtcgc gccgcgcaac acctggtaag 360
atggctgtgc gctcagatat atatagtgat atgcactaca aagatcataa ctagaccgcc 420
gcctcccccc ccccccctct ctaccttctc tctttctttc tccgtttttt ttttccgtct 480
cgtctcgatc tttggccttg gtagtttggg ggcgagaggc ggcttcgtcg cccagatcgg 540
tgcgcgtttt tttatttgga ggggcgggat ctcgcggctg ggtctcggcg tgcggccgga 600
ttctcgcggg gaatggggct ctcggatgtg gatctgatcc gccgttgttg ggggagatat 660
ggggcgttta aaatttcgcc atgctaaaca agatcaggaa gaggggaaaa gggcactatg 720
gtttaatttt tatatatttc tgctgctgct cgtcaggatt agatgtgctt gatctttctt 780
tcttcttttt gtgggtagaa tttgaatccc tcagcattgt tcatcggtag tttttctttt 840
gtcgatgctc accctgttgt ttggtgtttt tatactagtg gctatcctga cacggtctct 900
ttgtcaaata tctctgtgtg caggtataac tgcaggaaac aaattgaaca tcattctatc 960
aatacaacac aaacacaaca caactcaatc atttatttga caacacaact aaacaaccat 1020
ggtctagagc tc 1032

## Description

### Field of the invention

This invention is related with biotechnology and more specifically with Plant Genetic Engineering. In particular, chimerical DNA constructs are given, which shows a high transcription/translation promoter activity of any nucleotide sequence fused to them in dicot and monocot plant cells, which allows obtaining transgenic plants with higher expression levels of genes and DNA sequences of interest.

### Previous Art

Plant genetic engineering is a technology that has demonstrated to be very productive for basic investigation and commercial production of new biotechnological products. (Hammond J. Curr. Top. Microbiol. Immunol 1999, **240**:1-19; Simoens C. and Van Montagu M. Reproduction Update 1995, **1**:523-542).

The selection of promoter signals that warranty the adequate expression in terms of strength and temporal or spatial specificity of gene or DNA sequence introduced in plant genetically manipulated by the means of molecular biology techniques is very importat for the success of plant genetic engineering. That is why in the last two decades multiples efforts have been dedicated to the search of promoters and signals able to ensure the expression each transgene required. Thus, promoters of different origin (plant, viral, Ti or Ri *Agrobacterium* or chimerical) have been evaluated and employed in transgenic plant production.

The constitutive promoters more widely used in plant genetic manipulation have been the Cauliflower Mosaic Virus (CaMV) 35 S ARN promoter (Odell J.T; Nagy F; Chua N.H. Nature 1985, **313**:810-812); nopaline synthetase gene (nos) promoter from *A. tumefaciens* Ti plasmid (An G; Costa M.A; Mitra A; Ha S; Marton L. Plant Phisiol. 1986, **88**:547-552), rice actin-1 gene promoter (McElroy D; Zhang W; Cao J; Wu R. Plant Cell 1990, **2:**163-171) and maize ubiquitin-1 gene promoter (Christensen A.H; Sharrock R.A; Quail P.H. Plant Mol. Biol. 1992, **18**:675-689). However, each of these natural expression systems have limitations, mainly because its expression levels are not enough high in any class of plants; for example, the promoter expression is low in dicot plant cells and almost undetectable in monocots, while the expression of CaMV 35 S, the most widely used promoter, is much stronger in tobacco cells than in monocot plants (Topfer R; Maas C; Horicke-Grandpierre C; Schell J; Steinbiss H.H. Methods Enzymol. 1993, **217**:67-78; Mitsuhara I; Ugaki M; Hirochika H; Ohshima M; Murakami T; Gotoh Y; et al. Plant Cell Physiol. 1996, **37**:49-59). Similarly, rice actin-1 and maize ubiquitin-1 promoters are highly efficient promoting transcription of its downstream genes in monocot plant cells, but its promoter activity in tobacco cells is low (Schledzewski K; Mendel R.R. Transgenic Research 1994, **3**:249-255).

In order to increase heterologous protein expression levels in transgenic plants, a variety of chimerical promoters where natural promoters are combined with transcription or translation enhancers have been designed. Among these enhancer elements we can mention Omega translational enhancer from Tobacco Mosaic Virus (TMV) (Gallie D.R; Sleat D.E; Watts J.W; Turner P.C; Wilson T.M.A. Nucleic Acids Res. 1987, **15**:3257-3273 ), translational enhancer from Tobacco Etch Virus (TEV) (Carrington J.C; Freed D.D. J. Virol. 1990, **64**:1590-1597), promoter transcriptional enhancers from octopine synthase (Fromm H; Katagiri F; Chua N.H. Plant Cell 1989, 1:977-984), mannopine synthase (Comai L; Moran P; Maslyar D. Plant Mol Biol. 1990, **15**:373-381) and CaMV 35S promoter (Kay R; Chan A; Daly M; McPherson J. Science 1987, **236**:1299-1302); and natural exons and introns, e.g., maize alcohol dehydrogenase intron 1 (Callis J; Fromm M; Walbot V. Genes Devel. 1987, **1**:1183-1200; Last D.I; Brettell R.I.S; Chamberlaine D.A; Chaudhury A.M; Larkin P.J; et al. Theor Appl. Gen. 1991, **81**:581-588), the first exon/intron from maize sucrose synthase (Maas C; Laufs J; Grant S; Korfhage C; Werr W. Plant Mol. Biol. 1991, **16**:199-207), the first exon/intron from rice Actin-1 gene (McElroy D; Blowers A.D; Jenes B; Wu R. Mol. Gen. Genet. 1991, **231**:150-160), etc. That gave rise to promoters like 2X35S, Mac, Emu and others (EP0459643; EP0651812), which are strong mainly in a specific class of plant cells, dicots or monocots. (Schledzewski K; Mendel R.R. Transgenic Research 1994, **3**:249-255).

The development of strong promoters which can be employed to express genes in both dicot and monocot plant cells has been and is a relevant topic of many laboratories, not just for the scientific challenge it represents or the savings that implies to have a unique genetic construction to transform diverse classes of plants, but also to have their own expression systems, which make easier biotechnological products production and commercialization. The synthetic promoter claimed in the patent application WO9943838 claimed in a sequence from the TATA box till the transcription initiation site with an elevated GC content (64 % or higher), fused in its 5' end to transcription enhancer sequences from 35S, maize ubiquitin-1 and octopine synthase promoters. In the other hand, in order to look for expression not only in dicot and monocot plants, but also avoiding sequence homologous-dependent gene silencing (Park Y.D; Papp I; Moscone E; Iglesias V; Vaucheret H; Matzke A; Matzke M.A. Plant J. 1996, **9**:183-194), patent application WO0058485 claims an artificial promoter derived from the combination of sequences coming from two plant viruses genomes: Commelina Yellow Mottle Virus (CoYMV) and Cassava Vein Mossaic Virus (CsVMV), and also enhancer sequences from 35S promoter.

Mechanisms permitting that different genetic elements enhance transcription or translation of nucleotide sequences are not still clear. For example, it has been reported that leader sequences from many RNA viruses can enhance translation of different messenger RNAs (mRNA), independently of the presence of cap (m⁷G (5') ppp (5') N) fused to the 5'end (Sleat D.E; Wilson T.M.A. 1992. Plant virus genomes as sources of novel functions for genetic manipulations. In: T.M.A. Wilson & J.W. Davies (Eds), Genetic engineering with plant viruses. CRC Press, Inc. p.55-113; Gallie D.R; Sleat D.E; Watts J.W; Turner P.C; Wilson T.M. Nucleic Acids Res. 1987, **15**:8693-8711). However, with the exception of the RNA secondary structure of all these viral leaders is not complex, is not determined another common element between its nucleotide sequences responding for its translational enhancer properties.

Particularly, it has been reported that translation enhancement of TMV Omega fragment is due to the presence of at least one copy of the octamer ACATTTAC, which is repeated in its sequence, and a 25-base (CAA)n region that is considered a critic motif (two copies of (CAA)n region are enough to confer high enhancer levels) (Gallie D.R; Walbot V. Nucleic Acids Res. 1992, **20**:4631-4638). However, a 28-base, CA-rich region from Potato X Virus (PVX) leader, have not shown translation enhancer activity "per se" (Pooggin M.M; Skryabin K.G. Mol. Gen. Genet. 1992, **234**:329-331), while it is reported that CCACC pentanucleotide present in the CA region of PVX leader, might have pairing interactions with the 3'end of 18S rRNA (Tomashevskaya O.L; Solovyev A.G; Karpova O.V; Fedorkin O.N; Rodionova N.P; Morozov S.Y; Atabekov J.G. J. Gen. Virol. 1993, **74**:2717-2724) It has been determined that some viral leaders have sequence elements involved in translation enhancer activities, such as the CCTTTAGGTT sequence conserved in carlavirus leaders like Potato Virus S (PVS) (Turner R; Bate N; Tewell D; Foster G.D. Arch. Virol. 1994, **134**:321-333) and the so-called internal control regions type 2 (ICR2) motif (GGTTCGANTCC), which is found in 27-base repeated regions in the RNA3 leader of Alfalfa Mosaic Virus (A1MV), needing two to reach optimal translation levels (van der Vossen E.A.G; Neeleman L; Bol J.F. Nucleic Acids Res. 1993, **21**:1361-1367).

In the case of the TEV leader two regions called CIRE-1 and CIRE-2, between nucleotides 28 to 65 and 66 to 118, respectively, have been identified as responsible for the translation enhancement properties of this 148 bp viral leader (Niepel M; Gallie D.R. J. Virol. 1999, **73**:9080-9088). However, inside CIRE regions it has not been defined an specific element considered critic for the enhancer activity of these viral leader.

As we referred above, introns of natural origin and its adjacent sequences have been also widely employed to enhance different gene expression systems, especially when the intron is near of the 5'end of the gene. However, an intron-mediated enhancement of gene expression (IME), depending on factors like intron origin, exonic flanking regions and cellular type, has been reported. A strong IME of the expression has been observed mainly in monocot plant cells, but in dicots it commonly does not exceed 2 to 5-fold. Molecular mechanisms behind IME have not been completely disclosed (Simpson G.G; Filipowicz W. Plant Mol. Biol. 1996, **32**:1-41; Schuler M.A. 1998. Plant pre-MRNA splicing. *In*: J. Bailey-Serres & D.R. Gallie (Eds), A look beyond transcription: mechanisms determining MRNA stability and translation in plants. American Society of Plant Physiologists. P. 1-19; Lorkovic Z.J; Kirk D.A.W; Lambermon M.H.L; Filipowicz W. Trends in Plant Science. 2000, **5**:160-167).

IME expression variations observed between monocot and dicot plant cells can be due to the known differences in the requirements needed for an adequate pre-mRNA processing in different classes plants cells. In fact, in monocot plant cells, but not in dicot plant cells, the presence of AU-rich segments in the intron sequence is not indispensable to its processing; monocot cells can process introns with high GC-content (more than 50 %) and complex secondary structures (hairpin-loops), which indicates that dicot plant cells are unable to process introns with complex secondary structures (Goodall G.J; Filipowicz W. The EMBO Journal 1991, **10**:2635-2644; Lorkovic Z.J; Kirk D.A.W; Lambermon M.H.L; Filipowicz W. Trends in Plant Science. 2000, **5**:160-167). These reasons can explain, at least partially, why current systems employing IME to artificially enhance nucleotide sequence expression, are class-specific.

### Detailed description of the invention

The expression promoter sequence proposed in this patent application provides a set of distinctive characteristics: 1) it is universally functional as it is active in dicot as well as in monocots plants cells, permitting the obtaining of transgenic plants of any class with high expression levels of the genes and DNA sequences of interest; 2) it is based on the combination of artificially assembled genetic elements, increasing mRNA levels not only by IME, but also promoting its translation; 3) the lack of long DNA fragments with identical sequence to natural or viral genes in this promoter minimize the risk of RNA-mediated homologous gene silencing and the possibility of the appearance of new viral races or strains as a result of *in planta* homologous recombination; 4) the GC content of the sequence from the TATA box to the transcription initiation site must not necessarily to be high; 5) the versatility of our promoter sequence permits to insert in its 5' end transcription regulatory elements, which confers temporal, organ or tissue-specificity to the expression; 6) artificial genetic elements comprising it can be also functionally inserted between any promoter active in plant cells and any DNA sequence to increase its transcription/translation.

Chimerical exon/intron/exon region design with a high enhancement activity of mRNA accumulation in any class of plant cells, and its functional integration with an artificial translation enhancer constitute two essential components of the current patent application, because these elements permits us to efficiently express any DNA sequence of interest in plant cells.

It is important to clear that when we call a region, molecule or DNA sequence is artificial or chimerical, we are referring to its designed and synthesized *in vitro,* thus it is not any genetic element with identical primary DNA structure, although small fragments of its sequence could have a natural origin.

To design an intron with its correspondent adjacent exonic sequences, able to promote IME of expression, we studied which sequence motifs and genetic components were common to plant introns with reported transcription enhancer activity. At the same time, we had to resolve the challenge of achieving an adequate and efficient processing of this intron in dicot and monocot plants, independently of its GC content.

When the widely used as transcription enhancers promoters rice Actin-1, maize ubiquitin-1 and sucrose synthase-1 intron sequences are compared, it can be detected common and repeated sequence motifs in all of them (Figure 1). There is not demonstrated the responsibility of any of these motifs for the IME of gene expression these introns confers, but high conservation levels of the CTCC motif (or its homologous sequences CTC, TCC and TC) in these regions and in the 5' regions of the TATA boxes in a variety of plant promoters, indicates the possibility that it can favor the binding of transcriptional factors, which can promote RNA-polymerase II activity. At the same time, C and A-rich sequences abundance and conservation in the first exons (regions remaining as non translational mRNA untranslated leaders) and in the viral leaders with reported translational enhancer activity, indicates that such sequences can promote the stability of the resulting MRNA and its ability to be translated.

It is appropriated to emphasize that none of the above explained theories has a complete scientific demonstration, thus it is not obvious that the construction of an artificial intron with its adjacent exonic sequences, containing the repeated sequence motifs specified, results in a region that promotes high transcription levels and accumulation of mRNA; however, the results of our work indicates this.

From the mentioned comparison study between the different introns (with its correspondent exons), we decided to design an artificial exon/intron/exon region, which combines rice actin-1 and maize ubiquitine-1 intron/exon sequence fragments rich in the motifs that we consider relevant of the IME of gene expression. In order to achieve this goal, we had to take into account that the resulting artificial intron must be efficiently processed in dicot plat cells, so the increase of gene expression can take place in these class of plants too. Nevertheless, we find out that the intron sequences we used as prime material have a high GC content, complex secondary structures with abundant hairpin-loops, and the sequence of its AG acceptor 3' splicing site is some different from the branch point consensus sequence, thus these introns can be difficultly processed in dicot plant cells.

In order to simplify the secondary structure of the exon/intron/exon designed by us, so it can be processed in any class of plant cells, we decided to make some punctual changes in its sequence and to insert sequences UUUUUAU-like, which activates its processing (Gniadkowski M; Hemmings-Mieszczak M; Klahre U; Liu H.X; Filipowicz W. Nucleic Acids Res. 1996, **24**:619-627). Besides, our chimerical sequence was fused to the second exon and inserted into maize actin-1 gene second intron (IVS2), taking advantage of its efficient processing in dicot (e.g. tobacco) (Goodall G.J; Filipowicz W. The EMBO Journal 1991, **10**:2635-2644). The putative secondary structure of each artificial exon/intron/exon variant was studied by computing methods, using the PCFOLD 4.0 program (Zuker M. Meth. Enzymology 1989, **180**:262-288). The artificial exon/intron/exon sequence created for us will be called ART from this point.

As it was already mentioned, the second relevant component of this patent application is an artificial translational enhancer that was fused downstream to chimerical exon/intron in order to increase gene expression levels.

The artificial translational enhancer was designed from analysis of sequence and secondary structure formed on some viral leaders. From this analysis we conclude that there are three essential elements in the translational enhancers: 1) low complexity of secondary structures; 2) C and A-rich sequence segments; 3) motifs with up to 83% of homology with the consensus HCAYYY sequence (H= C ó U ó A; Y= C ó U, see Table 1), exposed and frequently repeated and/or in the hairpin of structures with a low melting point tails.

**Table 1.**

| Structurally conserved sequences in some RNA virus leader fragments (H=C/U/A; Y=C/U). | |
|---|---|
| Viral leader | Sequence |
| TMV | ACAUUUAC |
| TEV (CIRE-1) | GCAUUCUA |
| TEV (CIRE-2) | UCAUUUCU |
| PVS | ACCUUUAG |
| A1MV(RNA3) | UAAUUCG |
| A1MV(RNA3) | ACUUUUC |
| PVX | CCAAUUG |
| BMV | AACAUCGG |
| RSV | CCAUUCA |
| **Consensus** | **HCAYYY** |

From the premises above pointed out, we designed an artificial translational enhancer in which sequences HCAYYY-like, each one in hairpin-loop structures, were inserted inside a 45-base, C and A-rich sequence. This artificial translational enhancer have no more than 55 % of homology with RNA viral leaders which provided theoretical premises employed in its confection, and there is no even a sequence segment with more than 6 nucleotides with 100% of homology. That is why we can affirm that our translational enhancer is not a derivative of any translational enhancer previously reported or protected (EP 0270611), neither has sequences directly derived from them.

In order to make easier its manipulation and the fusion of genes of interest, restriction sites were added to our translational enhancer. Finally, before to fuse the new translational enhancer to the artificial Exon/Intron we created, its functionality was *in vivo,* showing the same capacity to enhance expression of a chimerical gene when compared with TMV Omega fragment. The artificial translational enhancer created for us will be called Eureka. (Figure 2).

To construct the promoter sequence claimed in this patent, a core promoter formed by a consensus TATA box (Joshi C.P. Nucleic Acids Res. 1987, **15**:6643-6653) was firstly designed which was fused to CaMV 35 S -24 to -4 region (from the transcription initiation site), followed by actin-1 -5 to +27 promoter region, which provides the transcription initiation site and a C and A-rich region. The maize ubiquitine-1 region, from +26 to +72 from the transcription initiation site, was fused downstream, providing AC- and TC-rich regions, yielding the first artificial exon, which was linked to maize actin-1 second exon, 12 bases before the 5' splicing site of its IVS2 intron and including itself. Bases change, adding or deletions were made around this joining according with the predictions the computing method in order to avoid putative secondary structures, which can affect RNA maturation. The artificial intron designed for us, is constituted by the first 54 bases of IVS2 intron, fused to 37 bases from a 5' region of the maize ubiquitine-1 first intron, corresponding to the bases +89 to + 126 from the transcription initiation site, followed by 375 bases from rice actin-1 first intron (from the position +103 to +477, from this gene transcription initiation site), fused to 33 bases from maize ubiquitine-1 intron 3' end (from the position +1051 to 1083 from the transcription initiation site), linked to the second half of the actin-1 IVS2 intron (from the position -52 to +5 from its 3' processing initiation site), and to a 29-base chimerical sequence containing restriction sites and a translation initiation consensus sequence. (Lütcke H.A; Chow K.C; Mickel F.S; Moss K.A; Kern H.F; Scheele G.A. The EMBO Journal. 1987, 6:43-48). The sequence of the artificial exon/intron/exon ART created for us is shown in figure 3. Once the efficient processing of the artificial exon/intron/exon constructed was tested by transient expression in both tobacco and rice cells, the translational enhancer Eureka was fused to its 3'end, appreciating in figure 4 the final structure of the promoter sequence object of this invention (PARTE promoter).

It must be highlighted that the enhancer element ART designed by us showed a higher efficiency as gene expression enhancer than the commonly used rice actin-1 gene first exon/intron/exon; Eureka fragment is an additional enhancer to its activity.

In this work it can be for the first time achieved two artificial, very efficient genetic elements, enhancing the expression of any DNA sequence in transgenic plant cells of any class, which demonstrate the validity of the theoretical precepts we are based on. It is also for the first time that an artificial promoter with an AT content lower than 52 % is adequately processed in dicot plant cells, promoting a high IME of the expression. The construction of a completely artificial, highly efficient translational enhancer, with low homology with ARN viral leaders, is also novel.

Different transcription enhancer sequences were fused 5' to the promoter sequence object of this invention. Thus, rice actin-1 region from -43 to -310 (from the transcription initiation site) was fused to the 5' end of the promoter PARTE, as shown in figure 5, to form the promoter region APARTE, which also has *as*-1-like transcription enhancer elements (Benfey P.N; Chua N.H. Science, 1990, **250**:959-966) and a 556-base fragment from the 5' region of the maize ubiquitin-1 promoter (from -299 to -855 from the transcription initiation site), to finally obtain the U3ARTE promoter, which structure is showed in Figure 6.

Many promoter sequence variants were constructed from the described genetic elements (see figure 7), and all of they demonstrate its functionality by the means of *in vivo* assays, proving the synergic effect over the gene expression all the enhancer and activator regions employed.

The *as*-1 element employed in our constructions as a transcription enhancer (see fig 6) has a innovative design, because it has less than 50 % of homology with the octopine synthase palindromic enhancer (Ellis J.G; Llewellyn D.J; Walker J.C; Dennis E.S; Peacock W.J. EMBO J. 1987, **6**:3203-3208; EP0278659), is not identical (less than 85 % of identity) to any of this type of sequence variants claimed in a study done by Ellis et al (Bouchez D; Tokuhisa J.G; Llewellyn D.J; Dennis E.S; Ellis J.G. EMBO J. 1989, **8**:4197-4204; USPat. 5,837,849) and the TGACG motifs found in it are found in an unique flank sequence context.

Although the rice actin-1 gene has been described and used to express different genes (McElroy D; Zhang W; Cao J; Wu R. Plant Cell 1990, **2**:163-171; WO9109948), it is important to emphasize that our work reveals the transcription enhancer activity of its 5' region and its use as heterologous expression enhancer. Similarly, although the use of the maize ubiquitine-1 5' transcription enhancer region has been claimed (EP0342926), the 556-base fragment employed for us does not contains the "heat shock" elements defined as essentials for this enhancer functionality (it is found between the positions -188 to -214 of this promoter sequence), thus it is original and does not obviate the transcription enhancer activity of the ubiquitine sequence used by us.

The PARTE promoter was also fused to a small, 214-base fragment corresponding to the -31 to -245 region from the transcription initiation site of the rice *gluB-*1 gene (Takaiwa F; Oono K; Kato A. Plant Mol. Biol. 1991, **16**:49-58) to form the new promoter region GARTE (fig 8) Transient expression assays demonstrate that the new artificial promoter GARTE is highly efficient to express DNA sequences in seeds endosperm.

Thus, we conclude that functional combination of these chimerical 5' transcription enhancers has novelty, and greatly useful to produce genetic elements allowing the achievement of high expression levels of DNA sequences in plant cells, independently of the class they belong to.

Obviously, other 5'regulator regions from different promoters can be *cys*-fused to the object of this invention in order to achieve high expression levels and/or to confer temporal, organ or tissue specificity to the expression.

For somebody experienced in genetic engineering techniques, what is here described would make possible the use ART and Eureka enhancers, in combination with any transcription promoter element in plant cells, to enhance transcription/translation of any DNA sequence in monocot or dicot plant cells. Similarly, theoretical precepts obtained from our observations, leading us to the design of ART and Eureka, can be employed by someone with experience in molecular biology techniques to construct new DNA expression enhancer sequences in plant cells.

Considering the rising development of plant genetic engineering in the last two decades, it is obvious that the promoter object of this invention, joined to any gene and a transcription terminator sequence, can be inserted in a plant cell genetic transformation vector, and by the means of the use of well-established, efficient techniques, obtaining transgenic plants able to express the gene of interest.

In this patent application, a genetic transformation vector refers to a DNA molecule (purified or contained inside a bacterial cell or a virus), which serves as a carrying vehicle to introduce in a plant cell any DNA fragment previously inserted in it.

### Description of the drawings.

Figure 1. Rice Actin-1 (Act), maize ubiquitine-1 (Ubi) and maize sucrose synthase (Shrun) gene sequences from the transcription initiation site. In uppercase is shown the first exon and in lowercase the first intron 5' region and the localization of the repeated and common sequence motifs are underlined.
Figure 2. Eureka artificial translational enhancer sequence, where its relevant elements and restriction endonucleases recognition sites are shown.
Figure 3. ART Exon/Intron/Exon artificial sequence, showing the origin of each of its component fragments (lowercase: artificial intron; the bases inserted to create UUUUUAU-like sequences are double-underlined; simply underlined are marked some relevant recognition sites for restriction endonucleases).
Figure 4. Primary structure of this invention object (PARTE promoter), showing the core promoter (lowercase italic) fused to the ART Exon/Intron/Exon region (intron bases in lowercase, exon's in uppercase) and to the artificial translational enhancer EUREKA. Some relevant recognition sites for restriction endonucleases are underlined; TATA box is double-underlined and translation initiation codon is in bold.
Figure 5. Primary structure of the APARTE promoter, showing rice actin-1 5' regulatory region (region from -43 to -310 from the transcription initiation site, in italics uppercase) fused to PARTE promoter (in italics lowercase the promoter, in lowercase the intron; in uppercase the exons). Underlined are marked some relevant recognition sites for restriction endonucleases; TATA box is double-underlined and the translation initiation codon is in bold.
Figure 6. Primary structure of the U3ARTE promoter, showing its component elements: - 299 to -855 region from the maize *ubi-*1 gene transcription initiation site, in uppercase; *as*-1-like transcription enhancer, in bold uppercase; region from -43 to -310 from the transcription initiation site of the rice *act-*1 gene, in italics uppercase; PARTE promoter in lowercase (TATA box is double underlined, ART intron is in italics and the translation initiation site is simple underlined).
Figure 7. Promoter variants with the enhancer elements object of this invention. A: 35SEureka; B: 35SART; C: 35SARTE; D: PARTE; E: APARTE; F: 2A1PARTE; G: 2APARTE; H: U3ARTE. 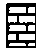 35S Promoter (1.3Kb); 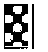 Translational enhancer Eureka; 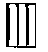 ART Exon/Intron/Exon; 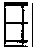 artificial promoter core; 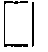 rice actin-1 gene 5' activation region (-43 a -221); 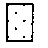 rice actin-1 gene 5'activation region (-226 a -310); 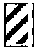 ASP *(as-*1-like enhancer); 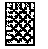 maize ubiquitine-1 promoter 5'activation region (-299 a -855).
Figure 8. Primary structure of GARTE promoter, showing its component elements: rice *glu*B-1 gene region from -31 to -245 from the transcription initiation site, in italics uppercase; PARTE promoter (promoter is in italics lowercase, intron in lowercase; exons are in uppercase; some relevant restriction sites are underlined; TATA-box is double underlined; translation initiation codon is in bold).
Figure 9. pUC-GUSint map.
Figure 10. pBPFΩ (omega) 7 map.
Figure 11. pBPFA 19-linker map.
Figure 12. Comparative demonstration by X-Gluc hystochemical dyeing of ART and EUREKA elements functionality in rice cells by the means of transient expression of different genetic constructions harboring GUSint gene, introduced by accelerated microprojectils bombardment.

### Microorganism Deposits

The plasmids pC-EURGUSint; pC-ARTEGUSint; pGARTEGUSint y pC-U3ARTEGUSint were deposited under the Budapest treated for the protection of Microorganisms in the Belgian Coordinated Collection of Microorganism, Plasmid Collection (BCCM/LMBP) Universiteit Gent, 'Fiers-Schell-Van Montagu' building, Technologiepark 927, B-9052 Gent-Zwijnaarde, Belgica. Plasmids pC-EURGUSint; pC-ARTEGUSint; pGARTEGUSint with the access numbers LMBP 4727; LMBP 4725; LMBP 4728 respectively and with the date May 19, 2003 and pC-U3ARTEGUSint with the number 4791 of November 25, 2003.

### EXAMPLES

### Example No.1: Construction of the constituting elements of a new chimerical system for the expression of DNA sequences in plant cells

All the synthesized DNA fragments were created with sticking ends to different type-II restriction endonucleases restriction sites in order to make easier its correct cloning.

### a) Eureka translational enhancer cloning.

The 86 base pairs (bp) DNA fragment corresponding to translational enhancer EUREKA (SEQ ID NO: 1), was cloned into the vector pBluescript II SK (Stratagene, USA) previously digested with the restriction enzymes *Pst* I and *Sac* I, taking advantage of the sticking ends for both enzymes included in the design of the synthetic DNA fragment. The resulting plasmid was named pBS-Eureka.

### b) Assemble of the artificial Exon/Intron/Exon region ART.

The artificial Exon/Intron/Exon region ART was constructed by cloning, assembling, one behind the other, DNA fragments designed. Firstly, the DNA synthetic fragment named P35AcU (SEQ ID NO: 2), which contains the core promoter, the first Exon and part of the artificial Intron, was cloned into the pBluescript II SK vector digested with *Eco* RI and *Spe* I restriction enzymes to obtain the plasmid pBS-AcU. After that, such plasmid was digested with *Spe* I and *Sac* I, inserting in it the DNA synthetic fragment I-U/Ac (SEQ ID NO: 3), which codes for part of the artificial intron. That is the way the pBS-AcUAc plasmid was obtained.

Next, the DNA synthetic fragment 1-Ac/U (SEQ ID NO: 4), harboring the end of the artificial Intron, was inserted into the pBS-AcUAc plasmid digested with the restriction enzymes *Bam* HI and *Sac* I, to produce the plasmid pBS-AcUAcU.

When the fragment IniT (SEQ ID NO: 5) was inserted into the *Spe*I / *Sac*I-digested pBS-AcUAcU, the artificial Exon/Intron/Exon ART was completed (SEQ ID NO: 6), conforming the plasmid pBS-ART, which primary structure between the restriction sites *Eco*RI and *Sac*I of the pBluescript II vector is shown in the sequence SEQ ID NO: 7.

### c) PARTE promoter construction.

To construct the promoter sequence object of this invention (PARTE promoter), the DNA fragment containing the core promoter and the Exon/Intron/Exon region ART (without its 3' region) was obtained from pBS-ART plasmid by an *Xho*I / *Pst*I digestion, inserting it into the pBS-Eureka plasmid digested with the same enzymes. Thus, we achieved the plasmid pPARTE (Figure 4, Figure 7D), which sequence between the *Eco*RI and *Sac*I sites is shown in sequence SEQ ID NO: 9.

### Example No.2: Demonstration of Eureka and ART enhancer elements functionality in plant cells.

### a) Translational enhancer Eureka functionality in tobacco cells.

To verify the enhancer power of Eureka in tobacco and rice cells, a series of auxiliary genetic constructions was made.

The reporter gene *uid*A with potato ST-LS1 gene IV2 intron inserted into the *Sna* BI site (GUSint), was obtained by an *Nco* I / *Sac* I digestion of plasmid pUC-GUSint (Figure 9) and cloned in the same sites of plasmid pBS-Eureka, giving rise to the vector Pbs-EURGUSint. This one was further digested with the restriction enzymes *Pst*I*, Sal*I and treated with S-1 Nuclease to obtain plasmid pBS-ΔEURGUSint; which was *Xho* I / *Kpn* I digested to obtain a DNA fragment containing the Eureka enhancer fused to GUSint gene, that was inserted in the pBPFΩ (omega) 7 vector (Figure 10). Thus we obtained the pBPF-EURGUSint vector (Figure 7A), having GUSint gene expression under the control of CaMV 35S promoter (1.3 kb version), Eureka enhancer and *Agrobacterium tumefaciens nos* gene transcription termination signals (tNOS).

As a control to evaluate the expression of the construction pBPF-EURGUSint, the plasmid pBPFΩ(omega)-GUSint was constructed, cloning GUSint gene, obtained from the plasmid pUC-GUSint by a *Sal*I / Klenow and *Kpn*I digestion, between the pBPFΩ (omega) 7 vector *Sma*I and *Kpn*I sites. This plasmid is similar to pBPF-EURGUSint except for the presence of the translational enhancer Ω (omega) controlling GUSint instead of Eureka. Another control plasmid was constructed by eliminating the enhancer omega of plasmid pBPFΩ(omega)-GUSint by *Xho* I - *Nco* I digestion, treatment with Klenow and plasmid self-ligation, obtaining pBPF-GUSint vector.

Plasmids pBPFSΩ(omega)-GUSint, pBPF -GUSint, y pBPF-EURGUSint were *Hind*III digested to obtain the cassettes for GUSint expression in plants; these were cloned into *Hind*III*-digested* binary vector pCAMBIA2300, giving rise to binary vectors pC-Ω(omega)7GUSint, pC-GUSint y pC-EURGUSint, respectively.

Binary plasmids obtained were introduced into *A. tumefaciens* strain LBA4404, we proceed to assay functionality of the enhancer Eureka by the means of a transient expression experiment in NT1 tobacco cells, following the protocol described by An et al (An G. Plant Physiol. 1985, **79**:568-570) with some modifications. After four days co-culturing tobacco cells with *Agrobacterium* carrying each of the binary vectors, the cells were collected and processed as described by Jefferson (Jefferson R.A. 1988. Plant reporter genes: the GUS gene fusion system. *In*: J.K. Setlow (Ed), Genetic Engineering. Vol.10, Plenum Publishing Corporation. P.247-263) to determine its β-glucuronidase (GUS) activity. Each experiment was repeated three times, with 5 replicas per construction each time. The results are shown in the following table.

**Table 2.**

| Demonstration of the functionality of the translational enhancer Eureka in tobacco cells. | | | | | | |
|---|---|---|---|---|---|---|
| Experiment | GUS Activity (Pm 4-MU/min/mg total proteins) | | | | Eureka / Ω (omega) rate | Eureka / Ω (omega) rate media |
| | Cell control | pC-GUSint | pC-Ω (omega) 7GUSint | pC-EURGUSint | | |
| I | 0.31±0.01 | 1.93±1.17 | 7.15+2.26 | 7.50±2.60 | 1.04 | 1.00±0.33 |
| II | 1.10±0.28 | 2.11±0.18 | 8.56±1.60 | 11.22±2.80 | 1.31 | |
| III | 0.79±0.19 | 4.84±1.66 | 33.2±3.6 | 21.1±6.1 | 0.64 | |

As it can be seen in results showed in Table 2, there are not significant differences between enhancer activities in Eureka when compared with that of TMV Omega leader sequence, which demonstrate that it has been for the first time achieved a completely artificial, efficient translational enhancer. This also confirm our theoretical precepts, showing that it is possible to construct a genetic element with significant properties to enhance the translation of DNA sequences fused downstream in 3' direction end by combining regions of sequences rich in C and A with sequences homologous to the motif HCAYYY (H=C/T/A; Y=C/T).

### b) Functionality of the artificial Exon/Intron/Exon ART in tobacco cells.

To test the functionality of the ART element in tobacco cells, it was firstly obtained the GUSint fragment from plasmid pUC-GUSint digesting it *Nco* I *- Sac* I, and it was cloned into pBS-ART digested with the same enzymes, to obtain plasmid pBS-ARTGUSint. Next, this plasmid was *Sal*I *- Bgl* II digested and treated with S1-Nuclease, obtaining pBS-ΔARTGUSint, which was digested *Xho*I *- Kpn*I to obtain the ARTGUSint fragment, and cloned into the vector pBPFΩ(omega)7-GUSint digested with the same enzymes, obtaining the plasmid pBPFARTGUSint (Figure 7B), where GUSint expression is under the control of CaMV 35S promoter (1.3 kb version), the artificial Exon/Intron/Exon region ART and *A. tumefaciens nos* gene transcription termination signals (tNOS).

From the plasmid pBS-ΔARTGUSint was obtained by *Xho*I / *Pst*I digestion the band containing the element ART, which was fused into the pBS-EURGUSint vector digested with the same enzymes to obtain the plasmid pBS-ARTEGUSint. From this one was obtained by *Xho*I / *Kpn*I digestion a DNA fragment to get the GUSint gene under the signals of the genetic elements ART and Eureka, introduced into pBPFΩ(omega)7 vector equally digested to produce pBPFARTEGUSint plasmid (Figure 7C).

Plasmids pBPFARTGUSint and pBPFARTEGUSint were *Hind*III digested to obtain cassettes for GUSint expression in plants; these were cloned into the binary vector pCAMBIA2300, resulting in binary plasmids pC-ARTGUSint y pC-ARTEGUSint, respectively.

Following the introduction of the binary plasmids obtained into *Agrobacterium tumefaciens* strain LBA 4404, we proceed to assay functionality of the translational enhancer Eureka in transient expression assays, using NT1 tobacco cells as described in section (a) of this example. The results obtained are shown in Table 3.

**Table 3.**

| Demonstration of functionality of the genetic elements ART and Eureka in tobacco cells. | | | | | | |
|---|---|---|---|---|---|---|
| Experiment | GUS Activity (Pm 4-MU/min/mg total proteins) | | | | 35SART / 35S Rate | 35SARTE/35S Rate |
| | Cell Control | pC-GUSint | pC-ARTGUSint | pC-ARTEGUSint | | |
| I | 1.13±0.27 | 4.39±0.96 | 5.26±1.69 | 26.3±3.5 | 1.2 | 6.0 |
| II | 1.77±0.58 | 6.11±2.45 | 12.92±2.36 | 32.0±6.1 | 2.1 | 5.2 |
| III | 0.69±0.30 | 2.46±0.77 | 3.94±1.14 | 13.5±2.8 | 1.6 | 5.5 |
| Media±SD | | | | | 1.63±0.33 | 5.57±0.29 |

Results of ART functionality evaluation in tobacco cells revealed that the artificial intron is correctly processed and possess expression enhancer activity in dicot plant cells. Our experimental results also showed that positive synergism on expression levels obtained form the interaction between ART and Eureka elements in the construction pBPFARTEGUSint, where there is an 5-fold increase of the expression ability of the known natural promoter CaMV 35S.

It is also proved than the artificial genetic elements designed (ART and Eureka) can be functionally inserted between any promoter active in plant cells (CaMV 35S promoter, in this case) and any other DNA sequence (GUSint in our case) increasing its transcription/translation.

### c) Functionality of the enhancer elements Eureka and ART in rice cells.

A set of new constructions was made in order to prove functionality of our artificial enhancers in monocot plant cells. First, the *Xho*I *- Kpn* I fragment from the plasmid pBPFARTGUSint, harboring the *uid*A (*gus*) gene fused in its 5' end to the artificial Exon/Intron ART, was inserted into pBPFA19-linker vector (Figure 11) digested with the same restriction enzymes, to form the plasmid pBPFA19ARTGUSint. In this plasmid, rice actin-1 Exon/Intron/Exon region present in pBPFA19-linker has been substituted by the artificial element ART, remaining as the other regulatory elements the chimerical promoter A 19 (where quadruplicated octopine synthase *as*-1-like enhancer is fused to CaMV 35S promoter (400 bp version)) and the tNOS transcription terminator signal.

Similarly, the *Xho*I *- Kpn*I band from plasmid pBS-ARTEGUSint was cloned into the pBPFA19-linker vector to obtain the construction pBPFA19ARTEGUSint. A construction used as a control, pBPFA 19GUSint, was made by cloning GUSint fragment from plasmid pUC-GUSint into the *Nco*I / *Sac*I digested pBPFA19-linker vector. Another control plasmid used was pBPFΩ(omega)-GUSint.

Qualitative evaluation of the ability of ART and Eureka to enhance gene expression in rice cells was carried out by assaying transient expression in callus of variety indica Perla. Calli were obtained from mature seeds previously sterilized with sodium hipoclorite and alcohol, and cultured for 21 days in the dark in N6-2 media: N6 salts and vitamins (Chu C.C et al. Scientia Sinic 1975, **18**:659); 0.1g/L myo-inositol; 1 g/L casein hidrolisate; 2mg/L 2,4 D; 30g/L Sucrose; 3g/L Phytagel, Ph 5.7). The transformation was performed by micro-projectile bombardment: before the bombardment the calli were sub cultivated in N6-2 media supplemented with 0.4 M Manitol for osmotic pre-treatment. 1 µm spherical gold particles (BioRad) were used as micro-projectiles for bombardment following published protocols (Russell D.R., Wallace K.M., Bathe J.H., et al. Plant Cell Rep. 1993, **12**:165-169). Transformation was performed employing the PDS-1000/He system (BioRad). For the bombardment 30 callus were placed at the center of the plate and the conditions were: 1100 psi of pressure, at the distance of 6 cm, one shoot per plate. After bombardment, calli remained in the same osmotic media for 16 hours at the dark, to be then sub cultivated 2 days in N6-2 media without Manitol. GUS activity is revealed with X-Gluc by hystochemical method (Jefferson R.A. 1988. Plant reporter genes: the GUS gene fusion system. *In*: J.K. Setlow (Ed), Genetic Engineering. Vol.10, Plenum Publishing Corporation. P.247-263). Evaluation was performed by counting blue points and zones in each callus in a stereomicroscope (Figure 12). Table 4 shows the results obtained after 4 experiments with 3 replicas each.

**Table 4.**

| ART and Eureka functionality comparative demonstration in rice cells. | | | | |
|---|---|---|---|---|
| Experiment | Callus % with blue zones and points | | | |
| | pBPFΩ (omega)-GUSint | pBPFA19GUSint | pA19ARTGUSint | pA19ARTEGUSint |
| I | 40 | 60 | 100 | 100 |
| II | 43 | 80 | 93 | 97 |
| III | 33 | 70 | 87 | 90 |
| IV | 27 | 83 | 93 | 90 |
| Media±SD | 36±6 | 73±8 | 93±3 | 94±4 |

As it can be seen, these experimental results also confirm functionality of ART and Eureka as gene expression enhancer elements in monocot plant cells. It is important to highlight that in our assays, IME activity developed by the artificial Exon/Intron/Exon ART (pA19ARTGUSint), was higher than that observed for rice actin-1 gene first Exon/Intron/Exon (pBPFAI9GUSint), which is a genetic element with recognized gene expression enhancer ability. Additionally, although in our experiments a significant difference between the results obtained for constructions pA19ARTGUSint and pA19ARTEGUSint can not be appreciated, it is remarkable in figure 12 that the presence of Eureka fragment in the last construction strongly increases expression, because the size and intensity of blue colored zones after X-Gluc hystochemical staining of calli bombarded with pA19ARTEGUSint (Figure 12).

Concluding, the present example shows the functionality of the artificial genetic elements ART and Eureka as gene expression enhancers in any kind of plant cells. Besides, it was demonstrated that these enhancer elements are highly efficient, increasing expression levels independently of the promoter that they are fused to. Finally, it was also demonstrated that ART and Eureka could be combined for synergistically enhance even more the expression of downstream genes.

It is anew shown that ART and Eureka can be functionally inserted between any plant active promoter (e.g. A19) and any DNA sequence (GUSint gene) to increase its transcription/translation.

### Example No. 3: PARTE expression system variants employing different 5' transcription enhancer regions.

### a) Addition of rice actin-1 5' transcription activator region to PARTE promoter.

To *cys-*fuse the 5' transcription enhancer region from rice actin-1 gene to PARTE promoter, pPARTE plasmid was digested with the enzymes *Eco*RI and *Eco*RV*,* inserting in it the synthetic DNA fragment En-Ac1 (from -43 to -221 from the rice actin-1 gene transcription initiation site; SEQ ID NO: 10), with extremes that ligate with those enzymes. The resulting plasmid, pAIPARTE, was *Eco* RV and *Hind* III digested to insert the synthetic DNA fragment En-Ac2 (from -226 to -310 from the rice actin-1 transcription initiation site; SEQ ID NO: 11), completing actin-1 gene promoter 5'activator region and producing the plasmid pAPARTE (Figure 5, Figure 7E), which nucleotide sequence between the restriction sites *Hind*III and *Sac*I is shown in sequence SEQ ID NO: 12.

### b) Addition of as-1-like transcription enhancer sequences to APARTE promoter.

Plasmid pA1PARTE was *Nru*I and *Sal*I digested to insert in it the DNA synthetic fragment called ASP (SEQ ID NO: 13), which possessed sticking ends to the mentioned restriction enzymes and codifies for an *as*-1-like transcription enhancer sequence, producing the construction pASPΔA1PARTE. This plasmid was *Sal* I digested, treated with S1 Nuclease and then *Pst* I digested to obtain the approximately 900 bp DNA fragment later cloned into the vector pA1PARTE digested with the *Nru*I and *Pst*I*,* to finally obtain the plasmid pASPA1PARTE, which has the ASP enhancer inserted in the *Nru*I site into the rice actin-1 5' transcription activation region. By digesting it with the *Eco*RV *- Xho*I*,* a second ASP enhancer was inserted into the plasmid pASPA1PARTE, giving rise to the vector p2AIPARTE (in sequence SEQ ID NO: 14, the nucleotide sequence is shown between the restriction sites *Kpn*I and *Sac*I*),* and in Figure 7F its structure.

As it was explained above for pA1PARTE, an En-Ac2 fragment was also inserted into pASPA1PARTE to obtain the construction pASPAPARTE, where a second ASP enhancer was inserted by digesting it *Eco*RV *- Sal*I*,* to finally obtain the vector p2APARTE (Figure 7G). Its nucleotide sequence between the sites *Sal*I and *Sac*I is shown in SEQ ID NO: 15.

### c) U3ARTE promoter construction.

Firstly, it was amplified by Polymerase Chain Reaction (PCR), using the primers Oli-U1 (SEQ ID NO: 16) and Oli-U2 (SEQ ID NO: 17), an approximately 395 bp DNA fragment, corresponding to the region from -299 to -680 from maize *ubi-*1 gene transcription initiation site. The amplified fragment was digested with the restriction enzymes *Kpn*I and *Xho*I (both sites were included inside the primer) and cloned into similarly processed pBluescript II SK vector, obtaining the construction pBS-Ubi1. After that, a synthetic DNA fragment (En-U2), which codifies for maize *ubi-*1 gene from -680 to -855 (sequence SEQ ID NO: 18), was cloned into the *Nco*I / *Kpn*I digested pBS-Ubi1 vector, resulting in the construction pBS-Ubi2, which contains maize ubiquitine-1 gene 5' activator region (from -299 to -855, sequence SEQ ID NO: 19).

The 5' transcription activator cloned region from maize ubiquitine-1 gene does not contain the "heat shock" box was obtained from the plasmid pBS-Ubi2 by an *Xho* I *- Kpn* I digestion, and *cis*-inserted 5' to promoter 2APARTE by *Sal*I *- Kpn*I digestion of the vector, to obtain the so called construct pU3ARTE (Figure 6, Figure 7H). The sequence of the vector pU3ARTE between the sites *Kpn* I and *Sac* I is shown in sequence SEQ ID NO: 20.

### d) GARTE promoter construction.

To demonstrate the plasticity of the present invention object, we decided to fuse 5' regulatory regions from promoters with organ, tissue or development-specificity, which conferred high expression levels with the mentioned characteristics. Thus, rice *glu*B-1 gene 5' regulatory region, which controls gluteline organ specific-expression, was fused in *cys* and 5' to PARTE promoter. To achieve this goal, it was synthesized a 214 base pairs fragment (GLU), corresponding to the -31 to -245 region from *glu*B-1 transcription initiation site (SEQ ID NO: 21) and convenient cloning sites, to insert it into the *Eco*RI and *Xho*I digested pPARTE plasmid, producing the pGARTE vector (Figure 8), which primary structure between *Xho*I and *Sac*I sites is shown in the sequence SEQ ID NO: 22.

### Example No.4: Functionality of the different variants of the PARTE expression system in tobacco and rice cells.

To evaluate the expression levels of each promoter variant object of the present invention in monocot and dicot plant cells, it was deleted by a *Sma*I *- Spe*I digestion the CaMV 35S promoter controlling GUSint expression in the binary vector pC-ARTE-GUSint, inserting in its place the *Kpn*I / S1 nuclease - *Spe*I fragment from constructions pAPARTE, p2A1PARTE, p2APARTE and pU3ARTE, to produce the new binary vectors pC-APARTEGUSint, pC-2A1PARTEGUSint, pC-2APARTEGUSint y pC-U3ARTEGUSint.

### a) Assays on tobacco.

Binary vectors pC-APARTEGUSint, pC-2A1PARTEGUSint, pC-2APARTEGUSint y pC-U3ARTEGUSint were introduced into *A. tumefaciens* cells to carry out transient expression assays experiments in NT1 tobacco cells, as described in Example 2 section (a). The control plasmid used, pC-GUSint, has GUS expression controlled by CaMV 35S promoter (1,3 kb version) and tNOS terminator. Experiment were performed twice (five replicas each treatment) and results are shown in the following table:

**Table 5.**

| Functionality of the different variants of PARTE expression system in tobacco cells. | | | | | | |
|---|---|---|---|---|---|---|
| | GUS Activity (Pm 4-MU/min/mg total proteins) | | | | | |
| Experiment | Cell controls | pC-GUSint | pC-APARTE GUSint | pC-2A1PARTE GUSint | pC-2APARTE GUSint | pC-U3ARTE GUSint |
| I | 0.46±0.37 | 3.55±1.23 | 4.89±1.67 | 23.1±6.9 | 28.4±5.8 | 29.2±6.1 |
| II | 1.30±0.81 | 7.02±2.78 | 6.63±4.26 | 24.7±4.2 | 21.0±4.3 | 32.6±9.0 |

It is evident that our results corroborate the functionality of different promoter variants in this invention object, also showing that it is possible to modulate its activity depending on the 5' transcription regulatory regions fused to PARTE. It must be highlighted that with our genetic constructions we reached superior expression levels in dicot plant cells than that achieved when expression is controlled by the natural promoter CaMV 35S.

### b) Assays on rice.

The binary vectors pC-APARTEGUSint, pC-2A1PARTEGUSint, pC-2APARTEGUSint y pC-U3ARTEGUSint were bombarded on rice calli as described in Example 2 section (c) in order to carry out a transient evaluation of the activity of the PARTE promoter different variants. The control plasmid, pAct1-F (McElroy D; Zhang W; Cao J; Wu R. Plant Cell 1990, **2**:163-171), has the *gus* gene expression under the control of the rice actin-1 gene promoter and the tNOS terminator. The expression cassette was extracted from these plasmid by *Kpn*I - *Xba*I digestion and inserted into the binary plasmid pCAMBIA 2300 digested with the same restriction enzymes to produce the vector pC-Act1F

The bombardment experiments were performed three times with three replicas for each construction to be evaluated. The results obtained are shown in the following table:

**Table 6.**

| Functionality of different variants of PARTE promoter expression system in rice cells. | | | | | |
|---|---|---|---|---|---|
| Experiment | % of calli with blue zones and dots | | | | |
| | pC-Act1F | pC-APARTE GUSint | pC-2A1PARTE GUSint | pC-2APARTE GUSint | pC-U3ARTE GUSint |
| I | 67 | 73 | 92 | 100 | 100 |
| II | 63 | 88 | 95 | 91 | 92 |
| III | 81 | 85 | 90 | 87 | 100 |
| Media±SD | 70±4 | 82±6 | 92±2 | 93±5 | 97±4 |

The results shown in this table certify the functionality of the different variants of PARTE promoter in monocot plant cells, achieving expression levels superior to that of the natural promoter of rice actin-1 gene. Therefore, usefulness of the object of the present invention as an efficient genetic tool to achieve high expression levels of DNA sequences placed in *cys* under its control is confirmed.

### c) Assays on rice seeds.

To evaluate tissue specificity of GARTE promoter in rice cell endosperms, the *Sal*I Klenow - *Pst*I fragment of about 2.5 Kb from pBPFARTEGUSint vector, containing the Eureka fragment fused to GUSint gene with nos terminator (tNOS), was cloned into pGARTE vector *Xba*I / Klenow - *Pst*I digested, giving as a result the construction pGARTEGUSint.

It was also constructed a control plasmid, where the GARTE promoter in pGARTEGUSint is substituted by *Xho* I - *Nco* I digestion, by a seed endosperm-specific, highly efficient rice gluteline B-1 promoter obtained from *Sal*I *- Nco*I digestion of pGEM-T-GluB-1 plasmid. Thus, we obtained pGluGUSint.

Evaluation of GARTE promoter and its comparison with that of GluB1, was carried out according to Y-S. Hwang (Hwang Y-S; McCullar Cass; Huang N. Plant Science. 2001, **161**:1107-1116) by bombarding immature endosperms (8-9 days after polinization) isolated from the ear cariopsis of greenhouse cultured rice variety indica Perla. Fluorometric assay to determine GUS activity in endosperms was performed according to Jefferson (Jefferson R.A. 1988. Plant reporter genes: the GUS gene fusion system. *In*: J.K. Setlow (Ed), Genetic Engineering. Vol.10, Plenum Publishing Corporation. P.247-263), 24 hours after bombardment with gold micro-particles covered by plasmidic DNA to be evaluated. The results of the GUS activity obtained in two independent experiments with 5 replicas each, are shown in Table 7.

**Table 7.**

| Functionality of GARTE promoter in rice seed endosperms. | | | |
|---|---|---|---|
| Experiment | GUS activity (Pm 4-MU/hr/mg total proteins) | | GARTE/GluB-1 rate |
| | pGluGUSint | pGARTEGUSint | |
| I | 34±9 | 79±22 | 2.3 |
| II | 27±5 | 52±13 | 1.9 |

These results confirm that the chimerical promoter GARTE, based on artificial elements designed for us, is highly efficient to express genes in seed endosperms; although GLU sequence inserted on the GARTE promoter is able to confer specificity to the expression, it 'per se' does not guarantee high levels, which depends also on other elements conforming the promoter (Takaiwa F; Yamanouchi U; Yoshihara T; Washida H; Tanabe F; Kato A; Yamada K. Plant Mol Biol. 1996,**30**:1207-1221).

The showed data reaffirm that the insertion of regulatory regions upstream to the element object of this invention permits its use to efficiently conduce the expression of any DNA sequence with development-, organ- or tissue-specificity. To somebody experienced in molecular biology, it is obvious that GluB-1 promoter regulatory sequences inserted into the GARTE promoter can be successfully substituted for regulatory sequences responding to biotic stress (pathogen attack, for example), abiotic factors (e.g., wounding, extremely high or low temperatures, salinity, drought, the presence of some chemicals), oxidative stress, different organ and tissue development stages, etc.

It is also evident that DNA sequences cloned under the regulatory regions object of this invention can be introduced into plant cells and stably inserted by the means of known biological or physic-chemical transformation methods and that, from these genetically modified cells it is possible to regenerate fertile plants in which DNA sequences will conveniently express according to the promoter variant which they are fused to. Thus, the present invention reveals its potentiality to contribute to the production of transgenic plants with greater levels of resistance to pests, diseases, a variety of stresses, greater agricultural yields or highly efficient producing compounds with medical or industrial applications, among other uses.

## Claims

1. An artificial promoter **characterized** for being a recombinant DNA molecule promoting expression in plant cells of any DNA sequence fused to its 3' end, comprising:
a) A 5' transcription regulator element followed by,
b) An artificial core promoter comprising a TATA box, a nucleotide sequence with a GC content lower than 64% and a transcription initiation site fused in its 3' end to,
c) A synthetic nucleotide sequence transcriptable but not translatable, conformed by a first chimerical Exon, an artificial Intron able to enhance the expression of genes fused to it in plant cells, and a second chimerical Exon with translation enhancement properties of any gene inserted downstream.

2. An artificial promoter according to Claim 1 **characterized** for being a recombinant DNA molecule where the 5' transcription regulation element is artificial.

3. An artificial promoter according to Claim 1 **characterized** for been a recombinant DNA molecule where the 5' transcription regulation element is homologous to a DNA sequence that naturally enhances and/or regulates gene expression in plant cells.

4. An artificial promoter according to Claim 3 which 5' transcription regulation element comes from rice actin-1 gene.

5. An artificial promoter according to Claim 4, where its 5' transcription regulation element comprises the region from -43 to -310 from rice actin-1 gene transcription initiation site.

6. An artificial promoter according to Claim 5 wherein the 5' transcription regulation element nucleotide sequence corresponds to sequence in SEQ ID NO: 10 or to a fragment of it.

7. An artificial promoter according to Claim 5 wherein the 5' transcription regulation element nucleotide sequence corresponds to sequence in SEQ ID NO: 11 or to a fragment of it.

8. An artificial promoter according to Claim 3 where its 5' transcription regulation element comes from maize ubiquitine-1 gene.

9. An artificial promoter according to Claim 8 wherein the nucleotide sequence comprises the region from -299 to -855 from maize ubiquitine-1 gene transcription initiation site.

10. An artificial promoter according to Claim 9 wherein the 5' transcription regulation element corresponds to sequence in SEQ ID NO: 19 or to a fragment of it.

11. An artificial promoter according to Claims 2 and 3 where its 5' transcription regulation element is an *as*-1-like transcriptional enhancer.

12. An artificial promoter according to Claim 11 where nucleotide sequence of as-1-like transcriptional enhancer is essentially identical to the sequence fragment corresponding to nucleotides 7 to 26 in SEQ ID NO: 13, or to the complementary sequence.

13. An artificial promoter according to Claim 3 where its 5' transcription regulation element comes from a viral promoter.

14. An artificial promoter according to Claim 13 where its 5' transcription regulation element comes from CaMV 35S promoter.

15. An artificial promoter according to Claims 2 and 3 wherein the 5' transcription regulation element controls gene expression in plant cells with development-, organ- or tissue-specificity.

16. An artificial promoter according to Claim 15 where its 5' transcription regulation element controls expression in seeds.

17. An artificial promoter according to Claim 16 where its 5' transcription regulation element comes from rice gluteline B-1 gene.

18. An artificial promoter according to Claim 17, where its 5' transcription regulation element comprises a fragment of the region from -31 to -245 from the rice gluteline B-1 gene transcription initiation site.

19. An artificial promoter according to Claim 18 wherein the 5' transcription regulation element corresponds to sequence in SEQ ID NO: 21 or to a fragment of it.

20. An artificial promoter according to Claim 15 wherein the 5' transcription regulation element controls gene expression in plant cells under biotic or abiotic stress.

21. An artificial promoter according to Claim 15 where its 5' transcription regulation element controls gene expression in wounded plant tissues.

22. An artificial promoter according to Claim 1 wherein the 5' transcription regulation region comprises 2 or more regulator elements from different origin operatively fused, which individually responds to any of the characteristics said on claims from 2 to 21.

23. An artificial promoter according to Claim 1 wherein the first Exon from the artificial Exon/Intron/Exon region comprises sequence motifs C and A rich.

24. An artificial promoter according to Claim 1 wherein the first Exon from the artificial Exon/Intron/Exon region comprises sequences wherein are frequently repeated the motif CTCC and/or its homologous sequences CTC, TCC and TC.

25. An artificial promoter according to Claim 1, which Intron from the artificial Exon/Intron/Exon region comprises sequences where the CTCC motif and/or its homologous sequences CTC, TCC and TC are frequently repeated.

26. An artificial promoter according to Claims from 23 to 25 wherein nucleotide sequence of the artificial Exon/Intron/Exon region corresponds to SEQ ID NO: 6 or to a fragment of it.

27. An artificial promoter according to Claim 1 wherein the second Exon from the artificial Exon/Intron/Exon region comprises sequence motifs with high C and A content.

28. An artificial promoter according to Claim 1 wherein the second Exon from the artificial Exon/Intron/Exon region comprises at least a sequence 83 % homolog with motif HCAYYY (H= C ó T ó A; Y= C ó T).

29. An artificial promoter according to Claims 27 and 28 wherein nucleotide sequence of the second Exon from the artificial Exon/Intron/Exon region corresponds to sequence in SEQ ID NO: 1.

30. An artificial promoter according to Claims from 23 to 29 wherein nucleotide sequence of the artificial Exon/Intron/Exon region corresponds to SEQ ID NO: 8 or with a fragment of it.

31. An artificial promoter according to any of the claims from 1 to 30, wherein nucleotide sequence of the artificial Exon/Intron/Exon region corresponds, at least partially, to sequence in SEQ ID NO: 20.

32. A DNA fragment from an artificial promoter according to Claims from 1 to 31 such that, when fused to any promoter functional in plants, contributes to enhance expression of DNA sequences controlled by said promoter.

33. An artificial promoter fragment according to Claim 32 able to enhance translation of genes fused to it.

34. An artificial promoter fragment according to Claim 33, comprising at least a sequence 83 % homolog with motif HCAYYY (H= C ó T ó A; Y= C ó T).

35. An artificial promoter fragment according to Claim 33 with sequence motifs C and A rich.

36. An artificial promoter fragment according to Claim 33 wherein the nucleotide sequence corresponds to that on SEQ ID NO: 1.

37. An artificial promoter fragment according to claims from 33 to 36 that contributes to enhance translation of mRNA's produced from the CaMV 35S promoter in plant cells.

38. An artificial promoter fragment according to Claim 32 corresponding to an Exon/Intron/Exon region.

39. An artificial promoter fragment according to Claim 38 wherein the first Exon comprises sequence motifs C and A rich.

40. An artificial promoter fragment according to Claim 38 wherein its first Exon comprises sequences where are frequently repeated the motif CTCC and/or its homologous sequences CTC, TCC and TC.

41. An artificial promoter fragment according to Claim 38 wherein its Intron comprises sequences where are frequently repeated the motif CTCC and/or its homologous sequences CTC, TCC and TC.

42. An artificial promoter fragment according to Claim 38 wherein nucleotide sequence corresponds with sequence in SEQ ID NO: 6.

43. An artificial promoter fragment according to Claim 38 wherein nucleotide sequence corresponds with sequence in SEQ ID NO: 8.

44. An artificial promoter fragment from claims 38 to 43 that contributes to enhance the expression of any gene under the control of CaMV 35S promoter in plant cells.

45. An artificial promoter fragment according to Claim 32 corresponding to an as-1-like transcriptional enhancer.

46. An artificial promoter fragment which nucleotide sequence is essentially identical to that of the fragment corresponding to nucleotides 7 to 26 in SEQ ID NO: 13, or its complementary sequence.

47. An artificial promoter fragment according to Claim 32 corresponding to a 5' transcription regulation element.

48. An artificial promoter fragment according to Claim 47 wherein the 5' transcription regulation element comes from rice actin-1 gene.

49. An artificial promoter fragment according to Claim 48 wherein nucleotide sequence comprises a fragment from -43 to -310 from rice actin-1 gene transcription initiation site.

50. An artificial promoter fragment according to Claim 49 wherein nucleotide sequence corresponds to sequence in SEQ ID NO: 10 or to a fragment of it.

51. An artificial promoter fragment according to Claim 49 wherein nucleotide sequence corresponds to sequence SEQ ID NO: 11 or to a fragment of it.

52. An artificial promoter fragment according to Claim 47 where its 5' transcription regulation element comes from maize ubiquitine-1 gene.

53. An artificial promoter fragment according to Claim 52 wherein nucleotide sequence comprises the region from -299 to -855 from maize ubiquitine-1 gene transcription start site.

54. An artificial promoter fragment according to Claim 53 wherein the 5' transcription regulation element corresponds to sequence in SEQ ID NO: 19 or to a fragment of it.

55. A cassette for the expression of DNA sequences in plant cells containing an artificial promoter responding to any of the claims from 1 to 31.

56. A cassette for the expression of DNA sequences in plant cells containing a transcription enhancer element functionally fused to a DNA fragment responding to any of the claims from 32 to 54.

57. A DNA vector for plant cell transformation comprising one of the expression cassettes **characterized in** claims 55 or 56.

58. A bacterial cell carrying vector on claim 57 and its descendants.

59. A plant cell transformed with vector on claim 57, and its descendants.

60. A plant cell according to Claim 59 expressing the DNA fragment under the control of the artificial promoter in the expression cassette introduced by the means of the vector described on claim 57.

61. A plant cell according to Claim 59 with the expression cassette **characterized** on claim 55 or 56 stably integrated into its genome.

62. A transgenic plant regenerated from the plant cell **characterized** on claim 61.

63. A transgenic plant according to Claim 62 expressing the DNA fragment under the control of the artificial promoter comprised into the expression cassette introduced by the means of the vector described on claim 57.

64. Transgenic plant descendants **characterized in** claim 63.

65. Plants according to Claim 64 being dicots.

66. Plants according to Claim 65 being Solanaceae.

67. Plants according to Claim 66 belonging to one of the following species: tobacco, tomato or potato.

68. Plants according to Claim 64 being monocots.

69. Plants according to Claim 68 being graminae.

70. Plants according to Claim 69 belonging to one of the following species: rice, sugarcane, maize, wheat or barley.

71. The purification or use of recombinant proteins produced by cells or plants according to Claims 60 or 63 as a result of the expression of the DNA fragments sited under the control of the artificial promoter comprised into the expression cassette introduced by the means of the vector described on claim 57.
